# FASCICULE DE BREVET EUROPEEN

(11) **EP 0 650 601 B1**
(45) Date de publication et mention de la délivrance du brevet: **09.04.1997**
(21) Numéro de dépôt: 93916009.9
(22) Date de dépôt: 16.07.1993
(51) Int. Cl.: G01T 1/203, G01T 1/161

(54) **APPAREIL DE DETECTION ET DE LOCALISATION DE MARQUEURS BIOLOGIQUES RADIOACTIFS**
VORRICHTUNG ZUR DETEKTIERUNG UND LOKALISIERUNG VON RADIOACTIVEN BIOLOGISCHEN MARKIERUNGEN
APPARATUS FOR SENSING AND LOCATING RADIOACTIVE BIOLOGICAL TRACERS

(30) Priorité: 17.07.1992 FR 9208882
(43) Date de publication de la demande: 03.05.1995
(73) Titulaire: DIMASON, 91940 Gometz-le-Chatel (FR)
(72) Inventeur: Popescu, Gheorghe Domitian, 91940 Gometz-le-Chatel (FR)
(74) Mandataire: Gutmann, Ernest
(86) Numéro de dépôt international: FR9300729
(87) Numéro de publication internationale: WO9402868

(56) Documents cités:
- EP-A- 0 284 542
- WO-A-91/19998
- FR-A- 2 637 088
- US-A- 4 788 436
- JOURNAL OF NUCLEAR MEDICINE vol. 29, no. 6 , Juin 1988 , NEW YORK USA pages 1101 - 1106 HICKERNELL ET AL. 'DUAL - DETECTOR PROBE FOR SURGICAL TUMOR STAGING'

## Description

La présente invention concerne un appareil de détection et de localisation de marqueurs biologiques radioactifs. Un tel appareil est destiné au repérage des rayonnement β et γ émis par les noyaux radioactifs couplés à des molécules injectées dans le corps humain avant une intervention chirurgicale.

Dans l'art antérieur, on connait notamment le brevet européen n° 284542 de la société NEOPROBE CORPORATION concernant un détecteur et localisateur pour l'émission de rayonnement à basse énergie.

Ce document décrit un appareil pour détecter et localiser des sources de rayonnement retenues par une tumeur dans l'environnement d'un corps présentant un niveau de rayonnement de fond, comprenant une sonde manipulable à la main et comportant un boîtier ayant une portion antérieure s'étendant jusqu'à une fenêtre pouvant être placée au voisinage de la source et une portion saisissable à la main, s'étendant à partir de la portion antérieure.

L'appareil de l'art antérieur comporte en outre un circuit détecteur dans le boîtier afin de produire des charges induites en réponse à une interaction du rayonnement avec ce détecteur et de fournir des signaux du détecteur correspondant à ces charges, des moyens de transmission pour transmettre ces signaux du détecteur, des moyens de traitement des signaux et des moyens avertisseurs répondant à des signaux d'attaque en émettant un signal de sortie perceptible d'une matière audible, en réponse aux signaux d'attaque qui lui sont appliqués.

Les moyens de traitement des signaux comportent un réseau d'analyse de niveau d'énergie pour évaluer les signaux du détecteur par rapport au bruit présenté par l'appareil et pour produire des signaux de sortie de données impulsionnels représentant le rayonnement, en excluant pratiquement tout le bruit, des moyens de sélection de plage pour produire une valeur de fonction de plage pour les signaux de sortie de données impulsionnels, cette valeur représentant la cadence des signaux de sortie de données impulsionnels représentant le niveau de rayonnement de fond, et des moyens de commande répondant aux signaux de sortie de données impusionnels en produisant d'une manière continue les cadences d'apparition de ces signaux, pendant des intervalles de temps prédéterminés, apparaissant successivement, dont la durée est sélectionnée pour permettre une mise à jour rapide, et répondant à chaque valeur discrète de la cadence produite et de la fonction de plage en produisant les signaux d'attaque ayant une fréquence génératrice d'un son lorsque la valeur de la cadence d'apparition est supérieure à la valeur de la fonction de plage, si bien que le signal de sortie perceptible d'une manière audible est variable, en réponse à ladite fréquence génératrice de son, en étant fonction des cadences d'apparition des signaux de sortie de données impusionnels se trouvant au-dessus du niveau de rayonnement de fond.

La sonde active d'un tel appareil présente un encombrement significatif, ce qui ne permet pas une utilisation satisfaisante dans tous les cas d'interventions.

On a également proposé dans l'art antérieur (brevet français n° 2575858) un convertisseur d'images associé à un faisceau de fibres optiques scintillantes.

Comme pour l'appareil précédent, l'encombrement ne permet pas une utilisation clinique optimale dans toutes les interventions.

Il est également connu du document WO-A-9113998 une sonde de détection du rayonnement β. Cependant, la sonde décrite utilise des plastiques scintillants encombrants pour détecter les rayonnements connectés à des fibres optiques, et l'interface entre les plastiques et les fibres est cause de chute de sensibilité.

Le document FR-A-2637088 concerne les fibres optiques scintillantes et préconise l'addition d'un soluté organique particulier (le biphenylbenzoxazole (PBBO)) pour éviter les fuites de lumière. Cette solution est onéreuse.

L'objet de la présente invention est de proposer un appareil pour la détection et la localisation de rayonnement β et γ émis par un traceur radioactif, de faible coût et compatible avec une utilisation des zones difficilement accessibles.

A cet effet, l'invention concerne plus particulièrement un appareil pour détecter et localiser des sources de rayonnements ionisants retenues par une tumeur dans l'environnement d'un corps du type comprenant une sonde manipulable à la main et comportant un boîtier comprenant un photodétecteur susceptible de produire une réponse à une interaction de signaux lumineux avec ce photodétecteur, des moyens de traitement des signaux électriques provenant dudit photodétecteur pour fournir des signaux d'attaque, et des moyens avertisseurs répondant auxdits signaux d'attaque en émettant un signal de sortie perceptible d'une matière audible ou visuelle, en réponse auxdits signaux d'attaque qui leur sont appliqués, caractérisé en ce que la sonde manipulable comporte au moins une fibre optique plastique scintillante enrobée d'un matériau en forme de gaine souple opaque, et comportant une extrémité avant et une extrémité arrière, l'extrémité avant étant fermée par un bouchon constitué du même matériau et l'extrémité arrière reliant ladite sonde audit photodétecteur pour convertir lesdits signaux lumineux émis par la fibre optique scintillante en signaux électriques.

L'appareil selon l'invention est composé d'un boîtier contenant l'ensemble des circuits électronique de traitement du signal, le photodétecteur, et l'alimentation. Ce boîtier peut être disposé en un endroit relativement éloigné du champ opératoire, contrairement au appareil de l'art antérieur qui intégrait dans la sonde au moins le photodétecteur. Le chirurgien manipule une sonde qui est constituée par l'extrémité d'une gaine souple renfermant la ou les fibres optiques remplissant une première fonction de guide de lumière et une seconde fonction de conversion des rayonnements β et γ en impulsions lumineuses. Cette gaine présente une grande souplesse, et un diamètre réduit, de quelque millimètres, ce qui permet l'accès en des endroits inaccessibles aux appareils de l'art antérieur.

De plus, il n'existe aucune liaison électrique entre le boîtier et la partie de la sonde susceptible de venir en contact avec le patient. De ce fait, la sécurité d'utilisation de l'appareil selon l'invention est renforcée par rapport aux appareils de l'art antérieur dont le photomultiplicateur logé dans l'extrémité de la sonde nécessite une alimentation en haute tension. Selon un mode de réalisation avantageux, le boîtier comporte un tube photomultiplicateur et des moyens de couplage optique d'au moins une fibre optique scintillante enrobée d'un gaine souple opaque dont l'extrémité constitue ladite sonde manipulable.

De préférence, la gaine opaque souple est stérilisable et contient un faisceau de 2 à 6 fibres optiques scintillantes d'un diamètre d'environ 1 millimètre. Selon un mode de réalisation avantageux, un obturateur est disposé entre le photomultiplicateur et le moyens de couplage optique du faisceau de fibres scintillantes.

Ce mode de réalisation évite de saturer le photomultiplicateur lors d'un changement de la sonde. Selon une variante, l'extrémité de la fibre plastique scintillante est solidaire d'un outil d'exploration.

Selon une autre variante, l'extrémité de la fibre plastique scintillante est solidaire d'un outil chirurgical.

Selon une autre variante, plusieurs fibres optiques scintillantes, isolées entre elles, forment un faisceau relié à un photomultiplicateur multicanaux. Ce mode de réalisation permet de faire de l'imagerie de la zone radioactive. Les extrémités de détection des fibres sont organisées en matrice de N x M points de détection.

L'invention sera mieux comprise à la lecture de la description qui suit, faisant référence aux dessins où:
- la figure 1 représente une vue en coupe de la partie détection, en position séparée ;
- la figure 2 représente une vue en coupe de la partie détection, en position d'utilisation ;
- la figure 3 représente une vue schématique de l'appareil selon l'invention.

La partie détection de l'appareil selon l'invention comporte un photomultiplicateur (1) qui transforme le signal lumineux émis par la fibre optique scintillante (2) en un signal électrique. Le photomultiplicateur est par exemple un photomultiplicateur commercialisé par la société PHILIPS sous la référence RTC XP191, d'un diamètre utile de 14 millimètres, possédant une photocathode bialcaline rubium et 10 étages d'amplification.

La fibre scintillante (2) est par exemple composée d'un scintillateur formé par un soluté unique dissous dans un solvant. Le soluté est à titre d'exemple du (biphenylyl-4)-phenyl-6 benzoxazole (PBBO). Sa concentration est de 4.10 ⁻³ gramme par gramme de solvant, ce dernier étant du polystyrène (n₁ = 1,59). Le coeur de la fibre est revêtue d'un film de poly-méthylméthacrylate (PMMA n₂ = 1,49). L'épaisseur du film est d'environ 3 % du diamètre pour les fibres à section circulaire, ou d'environ 2 % de la largeur pour les fibres à section carrée. De telles fibres sont en particulier commercialisées par la société KURABAY sous les références SCS-38 ou SCSF-81.

Les fibres scintillantes sont réunies en un faisceau de 4 fibres(2) entouré d'une gaine de protection (3) en une matière plastique opaque stérilisable sous atmosphère d'oxyde d'éthylène à 55 °C, par exemple le matériau commercialisé sous la dénomination ISOVERSINIC, d'une épaisseur d'un millimètre.

L'extrémité avant (4) du faisceau de fibres (2) est fermée par un bouchon (5) constitué en un matériau identique à celui formant la gaine (3). La longueur totale de la fibre est d'environ deux mètres.

Afin d'éviter l'éblouissement du tube photomultiplicateur (1) lors du remplacement de la fibre scintillante, celui-ci est logé dans une pièce tubulaire (6) dont les surfaces intérieures sont recouvertes d'un revêtement absorbant, par exemple une peinture noire mate. La pièce tubulaire (6) présente à son extrémité un embout de raccordement (7) complémentaire d'un bouchon (8) solidaire de l'extrémité (9) de la fibre (3).

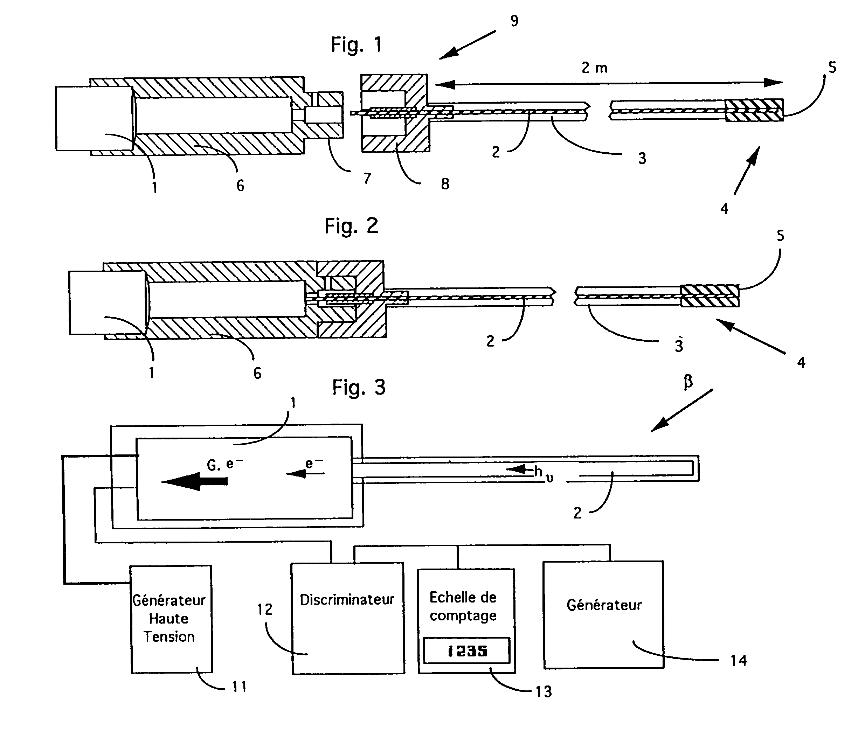

Le bouchon (8) vient se loger, comme représenté en figure 2, sur l'embout (7) de la pièce tubulaire (6) pour assurer le raccordement étanche de la fibre (2) sur le boîtier. Eventuellement, la pièce tubulaire (6) comporte un diaphragme isolant optiquement le photomultiplicateur (1) de la lumière extérieure lorsque la fibre (2) n'est pas raccordée sur l'embout (7).

La figure 3 représente une vue schématique de de l'ensemble de l'appareil selon l'invention.

Il comporte un générateur de Haute Tension (11) délivrant une tension d'alimentation du photomultiplicateur compris entre 1000 volts et 1800 volts.

Le signal provenant du photomultiplicateur (1) est traité par un discriminateur (12) qui effectue une comparaison avec une tension de référence réglable entre 20 et 120 millivolts dans l'exemple décrit. Le signal ainsi traité est transmis à un circuit de comptage (13), et commande par ailleurs un générateur sonore (14) provoquant un signal sonore à chaque impulsion détectée. Ce générateur permet à l'utilisateur de repérer la cible émettant des rayonnements ionisants de façon auditive, sans quitter du regard le champ opératoire.

L'invention est décrite dans ce qui précède à titre d'exemple non limitatif. Il est bien entendu que l'Homme du Métier sera à même de réaliser de nombreuses variantes sans sortir du cadre de la protection défini dans les revendications.

## Revendications

1. Appareil pour détecter et localiser des sources de rayonnements ionisants retenues par une tumeur dans l'environnement d'un corps du type comprenant une sonde manipulable à la main et comportant un boîtier comprenant un photodétecteur (1) susceptible de produire une réponse à une interaction de signaux lumineux avec ce photodétecteur (1), des moyens de traitement des signaux électriques provenant dudit photodétecteur (1) pour fournir des signaux d'attaque, et des moyens avertisseurs (13,14) répondant auxdits signaux d'attaque en émettant un signal de sortie perceptible d'une matière audible ou visuelle, en réponse auxdits signaux d'attaque qui leur sont appliqués, caractérisé en ce que la sonde manipulable comporte au moins une fibre optique plastique scintillante (2) enrobée d'un matériau en forme de gaine souple opaque (3), et comportant une extrémité avant (4) et une extrémité arrière, l'extrémité avant étant fermée par un bouchon (5) constitué du même matériau que la gaine et l'extrémité arrière reliant ladite sonde audit photodétecteur (1) pour convertir lesdits signaux lumineux émis par la fibre optique scintillante (2) en signaux électriques.

2. Appareil pour détecter et localiser des sources de rayonnement retenues par une tumeur selon la revendication 1, caractérisé en ce que le boîtier comporte un tube photomultiplicateur comme photodétecteur (1) et des moyens de couplage optique (6) d'au moins une fibre optique scintillante (2) audit photomultiplicateur.

3. Appareil pour détecter et localiser des sources de rayonnement retenues par une tumeur selon l'une quelconque des revendications précédentes, caractérisé en ce que la gaine opaque souple (3) est stérilisable et contient un faisceau de 2 à 6 fibres optiques scintillantes d'un diamètre unitaire d'environ 1 millimètre.

4. Appareil pour détecter et localiser des sources de rayonnement retenues par une tumeur selon l'une quelconque des revendications précédentes, caractérisé en ce qu'un obturateur est disposé entre le photomultiplicateur (1) et les moyens de couplage optique (6) du faisceau de fibres scintillantes.

5. Appareil pour détecter et localiser des sources de rayonnement retenues par une tumeur selon la revendication 1, caractérisé en ce que l'extrémité de la fibre plastique scintillante est solidaire d'un outil d'exploration.

6. Appareil pour détecter et localiser des sources de rayonnement retenues par une tumeur selon la revendication 1, caractérisé en ce que l'extrémité de la fibre plastique scintillante est solidaire d'un outil chirurgical.

7. Appareil pour détecter et localiser des sources de rayonnement retenues par une tumeur selon l'une quelconque des revendications 2 à 4, caractérisé en ce que le moyen de couplage optique est constitué par une pièce tubulaire (6) dans laquelle le photomultiplicateur (1) est logé, la pièce tubulaire (6) étant revêtue intérieurement d'un revêtement absorbant la lumière et présentant à son extrémité un moyen de raccordement (7) d'un connecteur prévu à l'extrémité de la fibre optique (2).

## Patentansprüche

1. Gerät zum Feststellen und Orten von innerhalb eines Körpers durch einen Tumor zurückgehaltenen Quellen ionisierender Strahlung, von der Bauart mit einer Sonde, die manuell handhabbar ist und ein Gehäuse aufweist, welches einen Photoempfänger (1) enthält, der im Stande ist, eine Reaktion auf eine Einwirkung von Lichtsignalen auf diesen Photoempfänger (1) zu erzeugen, mit Mitteln zum Verarbeiten von dem genannten Photoempfänger abgegebener elektrischer Signale, um Eingangssignale zu liefern, und mit Anzeigemitteln (13, 14), die auf die genannten Eingangssignale ansprechen, indem sie als Reaktion auf diese ihnen zugeführten Eingangssignale ein hör- oder sichtbares Ausgangssignal abgeben,
dadurch gekennzeichnet, daß die handhabbare Sonde mindestens eine scintillierende optische Plastikfaser (2) aufweist, die mit einem lichtdichten Werkstoff in Form einer Hülle (3) umkleidet ist und ein vorderes Ende (4) sowie ein hinteres Ende aufweist, von denen das vordere Ende mit einem Stopfen (5) verschlossen ist, der aus demselben Werkstoff wie die Hülle besteht, und das hintere Ende die genannte Sonde mit dem genannten Photoempfänger (1) verbindet, um die genannten, von der scintillierenden optischen Faser (2) abgegebenen Signale in elektrische Signale umzuwandeln.

2. Gerät zum Feststellen und Orten von durch einen Tumor zurückgehaltenen Strahlungsquellen nach Anspruch 1, dadurch gekennzeichnet, daß das Gehäuse einen Photomultiplikator als Photoempfänger (1) sowie Mittel (6) zum optischen Ankuppeln mindestens einer scintillierenden optischen Faser (2) an den genannten Photomultiplikator aufweist.

3. Gerät zum Feststellen und Orten von durch einen Tumor zurückgehaltenen Strahlungsquellen nach einem beliebigen der vorangehenden Ansprüche,
dadurch gekennzeichnet, daß die lichtundurchlässige weiche Hülle (3) sterilisierbar ist und ein Bündel von 2 bis 6 scintillierenden optischen Fasern gleichmäßigen Durchmessers von ungefähr 1 Millimeter enthält.

4. Gerät zum Feststellen und Orten von durch einen Tumor zurückgehaltenen Strahlungsquellen nach einem beliebigen der vorangehenden Ansprüche,
dadurch gekennzeichnet, daß zwischen dem Photomultiplikator (1) und den Mitteln (6) zum optischen Ankuppeln des Bündels scintillierender Fasern eine Blende angeordnet ist.

5. Gerät zum Feststellen und Orten von durch einen Tumor zurückgehaltenen Strahlungsquellen nach Anspruch 1,
dadurch gekennzeichnet, daß das Ende der scintillierenden Plastikfaser an einem Untersuchungsinstrument befestigt ist.

6. Gerät zum Feststellen und Orten von durch einen Tumor zurückgehaltenen Strahlungsquellen nach Anspruch 1,
dadurch gekennzeichnet, daß das Ende der scintillierenden Plastikfaser an einem chirurgischen Instrument befestigt ist.

7. Gerät zum Feststellen und Orten von durch einen Tumor zurückgehaltenen Strahlungsquellen nach einem beliebigen der Ansprüche 2 bis 4,
dadurch gekennzeichnet, daß das Mittel zum optischen Ankuppeln von einem rohrförmigen Bauteil (6) gebildet ist, in dem der Photomultiplikator (1) angeordnet ist, wobei das rohrförmige Bauteil (6) innen mit einer lichtabsorbierenden Auskleidung ausgekleidet ist und an seinem Ende ein Mittel (7) zum Anschließen eines am Ende der optischen Faser (2) vorgesehenen Steckers aufweist.

## Claims

1. Apparatus for detecting and locating ionizing-radiation sources retained by a tumour in the environment of a body, of the type comprising a probe which can be manipulated by hand and including a casing comprising a photodetector (1) capable of producing a response to an interaction of light signals with this photodetector (1), means for processing the electrical signals coming from the said photodetector (1) in order to provide drive signals, and warning means (13, 14) which respond to the said drive signals by emitting an audibly or visually perceptible output signal in response to the said drive signals which are applied to it, characterized in that the manipulable probe comprises at least one scintillating plastic optical fibre (2), clad with a material in the form of an opaque flexible jacket (3) and having a front end (4) and a rear end, the front end being closed by a plug (5) consisting of the same material as the jacket, and the rear end connecting the said probe to the said photodetector (1) in order to convert the said light signals emitted by the scintillating optical fibre (2) into electrical signals.

2. Apparatus for detecting and locating radiation sources retained by a tumour according to Claim 1, characterized in that the casing comprises a photomultiplier tube as photodetector (1), and means (6) for optically coupling at least one scintillating optical fibre (2) to the said photomultiplier.

3. Apparatus for detecting and locating radiation sources retained by a tumour according to either of the preceding claims, characterized in that the flexible opaque jacket (3) is sterilizable and contains a bundle of from 2 to 6 scintillating optical fibres having a unitary diameter of approximately 1 millimetre.

4. Apparatus for detecting and locating radiation sources retained by a tumour according to any one of the preceding claims, characterized in that a shutter is arranged between the photomultiplier (1) and the means (6) for optical coupling of the bundle of scintillating fibres.

5. Apparatus for detecting and locating radiation sources retained by a tumour according to Claim 1, characterized in that the end of the scintillating plastic fibre is solidly attached to an exploration tool.

6. Apparatus for detecting and locating radiation sources retained by a tumour according to Claim 1, characterized in that the end of the scintillating plastic fibre is solidly attached to a surgical tool.

7. Apparatus for detecting and locating radiation sources retained by a tumour according to any one of Claims 2 to 4, characterized in that the optical coupling means consists of a tubular part (6) in which the photomultiplier (1) is housed, the tubular part (6) being internally lined with a light-absorbent coating and having at its end a means (7) for connection of a connector provided at the end of the optical fibre (2).
